# EUROPEAN PATENT APPLICATION

(11) **EP 4 400 026 A1**
(43) Date of publication of application: **17.07.2024**
(21) Application number: 22814501.7
(22) Date of filing: 07.09.2022
(51) Int. Cl.: A61B 1/00, A61B 1/12, A61B 1/253

(54) **INTRAORAL ENDOSCOPE**

(30) Priority: 07.09.2021 RU 2021126363
(71) Applicant: Medrobotech Ltd, Limassol, 3031 (CY)
(72) Inventor: LIPPGARDT, Ivan Georgievich, Moscow, 115404 (RU); SOLOVYKH, Evgenii Anatol'evich, Moscow, 125362 (RU); TSIMBALISTOV, Aleksandr Viktorovich, Sankt- Peterburg, 198510 (RU); ZADUROV, Sergey Igorevich, Moscow, 117534 (RU); OBRUBOV, Aleksandr Andreevich, gor. Likino- Dulevo, 142672 (RU); PRESNOV, Iurii Vladimirovich, Moscow, 115304 (RU); ZHIDKOV, Maksim Yur'evich, Moscow, 111033 (RU); RAKHOV, Aleksandr Yaroslavovich, g. Korolev, 141070 (RU); BLINKOV, Valentin Alekseevich, Moscow, 111401 (RU); LUKARIN, Kirill Vital'evich, g. Novokubinsk, 352241 (RU); LIPPGARDT, Anastasiya Ivanovna, g. Podol'sk, 142115 (RU); TERKULOVA, Elena Vadimovna, Moscow, 127422 (RU); SOLOVYKH, Anastasiya Evgen'evna, Moscow, 105094 (RU)
(74) Representative: Koudine, Andreï
(86) International application number: PCT/IB2022/058433
(87) International publication number: WO 2023/037270

(57) **Abstract**

The invention relates to an intraoral endoscope including a housing (1) comprising a shielding window (6) disposed on a working end (101) of the housing (1). An optical sensor (8) connected to a microprocessor (4) is disposed inside the housing (1), in front of said shielding window (6). A backlight (5) is disposed inside the housing (1). The housing (1) is connected by a tube (3) to a compressed air supply source disposed outside the housing (1). The housing (1) comprises an opening for supplying air to an outer surface of the shielding window (6).

According to the invention, a diffuser (7) is disposed inside the opening. Said diffuser (7) is adapted to produce an air jet which generates an air curtain and has an outflow velocity from 6 m/s to 50 m/s forming an angle from 0° to 15° with a plane of the shielding window (6).

## Description

The present invention relates to intraoral endoscopes including a housing, an optically transparent shielding window, an optical sensor connected to a microprocessor, a backlight, the housing having an opening in the housing for supplying air to an outer surface of the shielding window, and can be used in dental procedures in a dentist's office.

There is an intraoral endoscope known from the prior art, said intraoral endoscope including a housing having an optically transparent shielding window disposed near a working end of the housing disposed intraorally; inside the housing in front of the window there is an optical sensor connected to a microprocessor and a backlight; the housing is connected by an air supply tube to a compressed air supply source disposed outside the housing; the housing have an opening into the housing for supplying air on an outer surface of the shielding window, see description of the French Patent FR2979054A1, published in 2013.

This device in its technical essence is the closest to the claimed invention and is considered as a prototype. Thus, a device proposed in this description will be described in terms of differences from the prototype.

A disadvantage of this device is that although it supplies the compressed air to the outer surface of the shielding window, it cannot ensure that particles from an oral cavity will not come into contact with it during the dental procedure, and is only designed to remove an existing dirt. Indeed, during a dental procedure, various instruments, for example dental burs, can create hard fragments that fly at high speed and can cross the air and hit the surface of the shielding window.

The present invention mainly aims to offer an intraoral endoscope allowing prevention of the typical particles of contamination from getting to the surface of the shielding window during a preparation of living hard tissues of a patient, which is the stated technical objective.

To achieve this objective, a diffuser is disposed inside the opening in the housing for supplying air. The diffuser is designed with the possibility to produce an air jet which generates an air curtain and has an outflow velocity in an interval of from 6 m/s to 50 m/s which forms an angle of 0° to 15° with a plane of the transparent shielding window.

In addition from simply blowing on the transparent shielding window to protect it from fogging, these useful features make it possible to generate a powerful air curtain to protect said shielding window from contamination with solid particles and liquid droplets produced during a preparation of the patient's living tissues, that is, to protect the shielding window from getting any particles on external optical components.

There is a possible optional embodiment of the invention in which the diffuser is disposed near the shielding window at the side of the working end of the housing and is designed with the possibility to direct the airjet away from the end of the housing.

These useful features make it possible to propose an embodiment of the intraoral endoscope when the diffuser is disposed near the shielding window at the side of the working end of the housing, but the air jet is directed away from the end of the housing, which is essential, because the air flow, which is rather significant, will not affect the patient.

There is an optional embodiment of the invention in which the diffuser is disposed near the shielding window at the side opposite to the working end of the housing. Under these conditions, the housing has a protrusion at the working end side, said protrusion having a surface shape which turns the air jet to 120°-140°.

These useful features make it possible to propose again an embodiment of the intraoral endoscope when the diffuser is although disposed near the shielding window at the side opposite to the working end of the housing, but the air jet is directed away from the end of the housing, which is essential, because the air flow, which is rather significant, will not affect the patient.

In addition, due to the fact that the housing has a protrusion at the working end side, said protrusion having a surface shape which turns the air jet to 120°-140°, the formed jet consists of two echelons with differently directed flows. Such complex flow of the high-speed jets makes it possible to increase an efficiency of interception of flying particles by extending their range in velocities and densities. This additionally prevents contamination of the optics.

Other distinguishing features and advantages of the invention are readily apparent from the description below which includes for illustration but is not limited to the following features, with reference to the figures attached, where:
- figure 1 represents a cross-section of an intraoral endoscope according to a first embodiment of the invention,
- figure 2 represents a cross-section of an intraoral endoscope fragment, showing in greater detail major assemblies according to the first embodiment of the invention,
- figure 3 represents a cross-section of an intraoral endoscope according to a second embodiment of the invention,
- figure 4 a cross-section of an intraoral endoscope fragment, showing in greater detail major assemblies according to the second embodiment of the invention.

The figures indicates:
1 - housing,
101 - working end of the housing, located intraorally,
102 - housing protrusion for turning an air jet,
2 - housing fixer,
3 - air flow supply tube,
4 - microprocessor,
5 - backlight, a light-emitting diode light source,
6 - optically transparent shielding window,
7 - diffuser,
8 - optical sensor (e.g. camera),
9 - dental wedge,
10 - rigid frame of the wedge,
11 - soft coating of the wedge,
12 - elastic leading-in piece.

A compressed air supply source (compressor of a dental unit) is not shown in the figures.

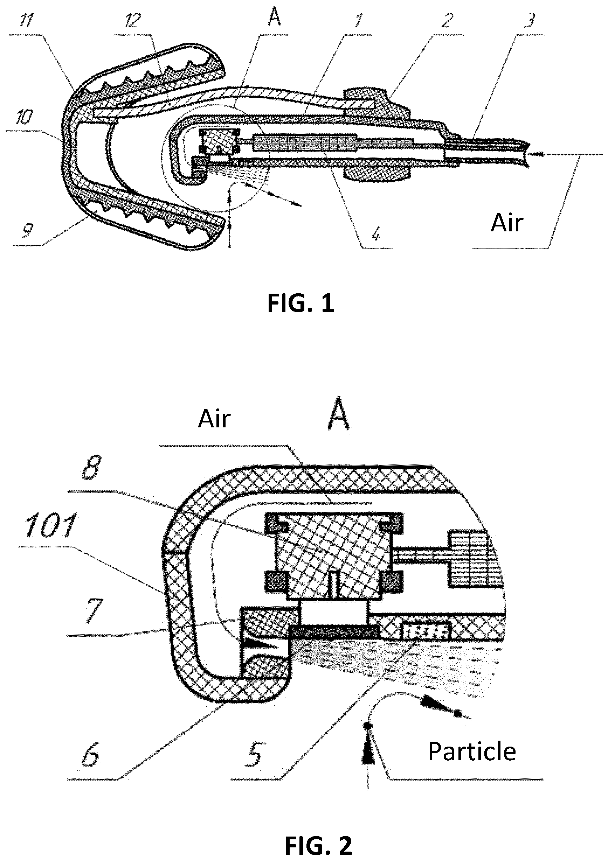

According to the Figures 1-4, an intraoral endoscope includes a housing 1 having an optically transparent shielding window 6 disposed near a working end 101 of the housing located intraorally. An optical sensor 8, connected to a microprocessor 4, is disposed inside the housing 1 in front of said window 6. As an example, the optical sensor 8 may comprise an optical camera, for example, a photo- and/or video- camera (preferably, a digital one). Inside the housing 1 there is a backlight 5. It is primarily a light-emitting diode light source. The housing 1 is connected by an airflow supply tube 3 to a compressed air supply source disposed outside the housing 1. The housing 1 has an opening in the housing for supplying air to an outer surface of the shielding window, inside which a diffuser is disposed.

A diffuser 7 is designed with the possibility to produce an air jet, which generates an air curtain and has an outflow velocity in an interval of from 6 m/s to 50 m/s forming an angle of 0° to 15° with a plane of the transparent shielding window 6.

The diffuser 7 may be disposed near the shielding window 6 at the side of the working end 101 of the housing and is designed with the possibility to direct the air jet away from the end of the housing: see Figures 1-2.

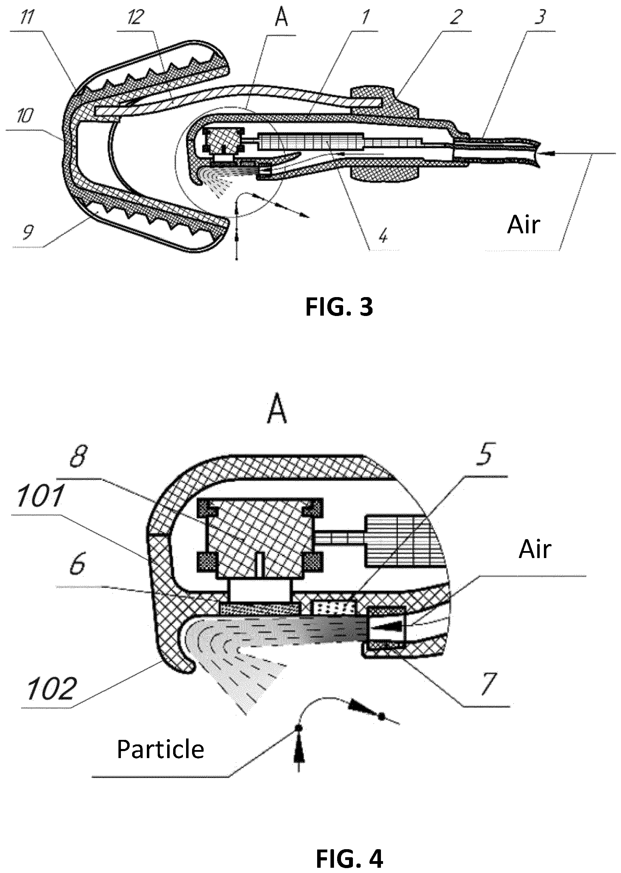

The diffuser 7 can be disposed near the shielding window 6 at the side opposite to the working end of the housing. In this (second) embodiment of the invention, the housing has a protrusion 102 at the working end side, said protrusion having a surface shape which turns the air jet to 120°-140°: see Figures 3-4.

In general, the intraoral endoscope may additionally include a basing dental wedge 9, which consists of a rigid wedge frame 10, an anti-slip soft cover of the wedge 11, a flexible and elastic leading-in piece 12 and a fixer 2 of the camera housing 8.

These elements allow the endoscope to be mounted on the patient.

Thus, the invention relates to an intraoral endoscope including a housing 1 with an optically transparent shielding window 6 disposed on a working end 101 of the housing 1, which is designed with the possibility to be located intraorally. In this case, an optical sensor 8 connected to a microprocessor 4 is disposed inside the housing 1 in front of the optically transparent shielding window 6. Also, a backlight 5 is disposed inside the housing 1. The housing 1 is connected by an air supply tube 3 to a compressed air supply source disposed outside the housing 1. In this case, the housing 1 comprises an opening (in the housing 1) for supplying air to an outer surface of the optically transparent shielding window 6.

According to the invention, a diffuser 7 is disposed inside the opening in the housing 1 for supplying air, said diffuser being designed with the possibility to produce an air jetthat generates an air curtain adapted to exclude an ingress of (external) particles onto the outer (i.e. directed out of the housing 1) surface of the optically transparent shielding window 6. In this case, the air jet has an outflow velocity (from the diffuser 7) comprising in an interval of from 6 m/s to 50 m/s (included) and forming a first angle with a plane of the optically transparent shielding window 6 (said plane being parallel to a longitudinal axis of the endoscope, coinciding with an arrow "Air" in Figures 1 and 3). Under these conditions, said first angle is included in a first interval of from 0° to 15° (included).

In a first embodiment of the invention, as shown in Figures 1-2, the diffuser 7 may be disposed at the side of the optically transparent shielding window 6 on the working end 101 of the housing 1. In the example in the figures 1-2 it is shown that the diffuser 7 is disposed along the longitudinal axis of the endoscope (coinciding with the "Air" arrow in figure 1) downstream of the air flow entering the endoscope housing 1 through the tube 3, with respect to the optically transparent shielding window 6. In this case, the diffuser 7 is designed with the possibility to direct the air jet away from the working end 101 of the housing 1.

In a second (different from the first) embodiment of the invention, as shown in Figures 3-4, the diffuser 7 may be disposed at the side of the optically transparent shielding window 6 on another end of the housing, said another end being opposite to the working end of the housing 1. In the example in the figures 3-4 it is shown that the diffuser 7 is disposed along the longitudinal axis of the endoscope (coinciding with the "Air" arrow in figure 3) upstream of the air flow entering the endoscope housing 1 through the tube 3, with respect to the optically transparent shielding window 6. In this case, the housing 1 comprises, at the side of the working end 101, a protrusion 102 having a surface shape which turns the air jet to a second angle included in a second range of from 120° to 140° (included).

Preferably, the backlight 5 is located along the longitudinal axis of the endoscope (coinciding with the "Air" arrow in figures 1-4) upstream of the air flow entering the endoscope housing1 through the tube 3, in relation to the optically transparent shielding window 6.

This beneficial feature contributes to a miniaturization of the intraoral endoscope according to the invention. Also, this beneficial feature ensures an optimal lighting of external objects examined with the help of the intraoral endoscope (e.g., patient's teeth) by a light flux coming from the backlight 5. In particular, this selective arrangement of the backlight 5 helps to ensure that a (returned) light flux (from the backlight 5) reflected from these external objects and passing through the optically transparent shielding window 6 towards the optical sensor 8 is sufficient for an expected operation of the optical sensor 8.

In the second embodiment of the invention mentioned above and shown in Figures 3-4, the diffuser 7 may be disposed along the longitudinal axis of the endoscope (coinciding with the "Air" arrow in Figure 3) upstream of the air flow entering the endoscope housing 1 through the tube 3 with respect to the backlight 5.

This beneficial feature contributes to the miniaturization of the intraoral endoscope according to the invention. Also, this beneficial feature ensures that the air jet produced by the diffuser 7 generates the air curtain adapted to exclude an ingress of external particles onto corresponding outer (i.e. directed out of the housing 1) surfaces of both the optically transparent shielding window 6 and the backlight 5. Such selective location of the diffuser 7, the backlight 5 and the optically transparent shielding window 6 relative to each other (along the longitudinal axis of the endoscope) provides the expected operation of the optical sensor 8 regardless of a current concentration of external particles in the patient's mouth due to use of a standard dental cutting tool.

The intraoral endoscope operates as follows. Below are the most exhaustive examples of the embodiment of the invention, however, said examples do not limit applications of the invention.

**Example 1.** As shown in Figures 1-2, air from a compressor of a dental unit is supplied to an intraoral endoscope housing 1 towards a microprocessor 4 and to an optical sensor 8 (camera) via an air supply tube 3 with an integrated cable conduit. Passing inside the housing 1 of the intraoral endoscope along the microprocessor (driver board) 4, this air flow effectively cools all heat-generating elements, which is additional technical result achieved.

After passing through the housing 1, the air enters a directing diffuser 7, which generates a flat jet directed at an angle from 1 degree to 15 degrees with a surface of the housing, with an outflow velocity of 6 m/s to 50 m/s. The high-speed jet does not allow any particles with densities and velocities typical for dental interventions to penetrate further than its core and draws them away from an optically transparent shielding window 6 of the optical sensor 8 (camera).

**Example 2.** As shown in Figures 3-4, air from a compressor of a dental unit is supplied to an intraoral endoscope housing 1 towards a microprocessor 4 via an air supply tube 3 with an integrated cable conduit. Passing inside the housing 1 of the intraoral endoscope along the microprocessor (driver board) 4, this air flow effectively cools all heat-generating elements, which is additional technical result achieved.

After passing through the housing 1, the air enters a directing diffuser 7 which generates a flat jet directed at an angle from of 1 degree to 15 degrees with a surface of the housing 1, with an outflow velocity of 6 m/s to 50 m/s. Said flat air jet, passing along an outer surface (directed out of the housing 1) of an optically transparent shielding window 6 gets in a special protrusion 102 on the housing 1, which turns it by 120-140 degrees angle. Thus, a jet consisting of two echelons with differently directed flows is formed. Such complex flow of the high-speed jets makes it possible to increase an efficiency of interception of flying particles by extending their range in velocities and densities.

In general, an operation of the intraoral endoscope according to the invention is possible in two modes: a fixation on an intraoral stand and a manual mode. The intraoral endoscope stand consists of a rigid basing element, which is a rigid-type jaw wedge 9. An elastic leading-in piece 12 which is realized in a ductile metal (copper, aluminum, steel, etc.) and is able to hold a weight of the endoscope (with a camera 8) in any position, is attached to the wedge 9.

On the other side, the leading-in piece is attached to a fixer 2 of the device, which allows a rotation of the housing 1 of the device, and the camera 8 with it, around a longitudinal axis of the endoscope (coinciding with the arrow "Air" in Figures 1 and 3), which allows a use of the intraoral endoscope for lower and upper jaws, without rearranging the basing wedge.

An intraoral endoscope may be implemented by a person skilled in the art in practice and ensures that the claimed objectives are met after implementation, which leads to the conclusion that the invention meets the requirement of "industrial applicability".

A series of preclinical interventions was performed on healthy adult volunteers to confirm an operability of this intraoral endoscope design under real conditions. As an example, a video recording of a preparation of an upper left 8th tooth with a carious lesion was made. The preparation was performed using a standard air-turbine hand piece with a standard water irrigation of an intervention area. A shielding window of a sensor was 25-28 mm away from the preparation area at 60-70 degrees angle. No additional optics protective measures were applied, only a standard dental equipment and instruments for similar procedures were used. The video shows that under conditions of active supply of cooling water, its highly intensive splashing in case of contact with the fast-rotating cutting instruments, a chipping of hard particles of enamel and dentin, there is no contam ination of the optically transparent protective window 6, and the optical sensor 8 (camera) continues to operate during an entire process of manipulation.

## Claims

1. An intraoral endoscope including a housing (1) comprising an optically transparent shielding window (6) disposed on a working end (101) of the housing (1), which is designed with the possibility to be located intraorally,
an optical sensor (8) connected to a microprocessor (4), said optical sensor (8) being disposed inside the housing (1), in front of the optically transparent shielding window (6),
a backlight (5) disposed inside the housing (1),
the housing (1) being connected by an air supply tube (3) to a compressed air supply source disposed outside the housing (1),
the housing (1) comprising an opening for supplying air on an outer surface of the optically transparent shielding window (6),
**characterized in that** a diffuser (7) is disposed inside the opening in the housing (1) for supplying air,
**in that** the diffuser (7) is designed with the possibility to produce an air jet which generates an air curtain and has an outflow velocity forming a first angle with a plane of the optically transparent shielding window (6), said outflow velocity being included in an interval of from 6 m/s to 50 m/s, and
**in that** said first angle is included in a first range of from 0° to 15°.

2. The intraoral endoscope according to claim 1, **characterized in that** the diffuser (7) is disposed at the side of the optically transparent shielding window (6) on the working end (101) of the housing (1), and
**in that** the diffuser (7) is designed with the possibility to direct the air jet away from the working end (101) of the housing (1).

3. The intraoral endoscope according to claim 1, **characterized in that** the diffuser (7) is disposed at the side of the optically transparent shielding window (6) on another end of the housing (1), said another end being opposite to the working end of the housing (1), and
**in that** the housing (1) comprises, at the side of the working end (101), a protrusion (102) having a surface shape which turns the air jet to a second angle, said second angle being included in a second range of from 120° and 140°.
